# EUROPÄISCHE PATENTANMELDUNG

(11) **EP 4 851 609 A1**
(43) Veröffentlichungstag der Anmeldung: **09.12.2026**
(21) Anmeldenummer: 25180474.6
(22) Anmeldetag: 03.06.2025
(51) Int. Cl.: G01N 21/51, G01N 21/64

(54) **HALTEVORRICHTUNG FÜR MINDESTENS EIN PROBEAUFNAHMEGEFÄSS UND MESSVORRICHTUNG ZUM QUANTITATIVEN BESTIMMEN MINDESTENS EINER FLUORESZIERENDEN, LICHTABSORBIERENDEN UND/ODER LICHTRÜCKSTREUENDEN KOMPONENTE**

(71) Anmelder: photonamic GmbH & Co KG, 25421 Pinneberg (DE)
(72) Erfinder: Szalata, Rainer, 85604 Zorneding (DE); Homann, Christian, 81369 München (DE); Eisel, Maximilian, 82362 Weilheim (DE)
(74) Vertreter: Hofstetter, Schurack & Partner

(57) **Zusammenfassung**

Die vorliegende Erfindung betrifft eine Haltevorrichtung (10) zur Aufnahme und Halterung von mindestens einem Probeaufnahmegefäß (12), wobei das Probeaufnahmegefäß (12) transparent und/oder transluzent ausgebildet ist und einen Aufnahmeraum (24) für ein zu untersuchendes, fließfähiges Medium aufweist, wobei die Haltevorrichtung (10) einen Halteblock (26) mit mindestens zwei, an einem Basiselement (28) angeordneten und parallel zueinander verlaufenden Halteblockschenkeln (30, 32) aufweist, wobei ein Zwischenraum zwischen den Halteblockschenkeln (30, 32) als Einführöffnung (36) für das Probeaufnahmegefäß (12) ausgebildet ist, und mindestens eine Beleuchtungseinrichtung (38, 40) zur Photooxidation des zu untersuchenden Mediums umfasst, wobei die Beleuchtungseinrichtung (38, 40) an einer in den Zwischenraum zeigenden Oberfläche von mindestens einem der Halteblockschenkeln (30, 32) direkt oder indirekt angeordnet ist, und ein dem Basiselement (28) des Halteblocks (26) gegenüberliegendes Ende des Zwischenraums als Lichteinlaßöffnung (34) für einen Anregungslichtstrahl (116, 118) ausgebildet ist. Die Erfindung betrifft weiterhin eine Messvorrichtung (70) mit einer derartigen Haltevorrichtung (10).

## Beschreibung

Die vorliegende Erfindung betrifft eine Haltevorrichtung zur Aufnahme und Halterung von mindestens einem Probeaufnahmegefäß, wobei das Probeaufnahmegefäß transparent und/oder transluzent ausgebildet ist und einen Aufnahmeraum für ein zu untersuchendes, fließfähiges Medium aufweist. Die Erfindung betrifft weiterhin eine Messvorrichtung zum quantitativen Bestimmen mindestens einer in einem fließfähigen Medium vorkommenden fluoreszierenden, lichtabsorbierenden und/oder lichtrückstreuenden Komponente.

Derartige Haltevorrichtungen und Messvorrichtungen sind aus dem Stand der Technik bekannt. So wird beispielsweise in Lang, A. et al.: "Rapid spectrophotometric quantification of urinary porphyrins and porphobilinogen as screening tool for attacks of acute porphyria," J. Biomed. Opt. 23(5), 055006 (2018), doi: 10.1117/1.JBO.23.5.055006 eine Messvorrichtung mit einer Haltevorrichtung für ein Probeaufnahmegefäß beschrieben, mit der Porphyrinwerte im menschlichen Urin gemessen werden können. Über entsprechende Lichtleiter wird ein Anregungslicht von einer Anregungslichtquelle zu der zu analysierenden Urinprobe geleitet. Zudem wird eine Photooxidation der Urinprobe mittels einer von außen, insbesondere von oben, über einen weiteren Lichtleiter zugeführten Lichtstrahlung vorgeschlagen. Erhöhte Werte an Porphyrinen im Urin sind üblicherweise Indikatoren verschiedenster Krankheiten. Nachteilig an der in Lang et al. dargestellten Messvorrichtung ist deren relativ komplizierter technischer Aufbau, da hier jeweils Lichtleiter durch eine Haltevorrichtung für ein Probeaufnahmegefäß zu diesem herangeführt werden müssen. Zudem erfolgt die Photooxidation über einen relativ kleinen Öffnungsquerschnitt des Probeaufnahmegefäßes, so dass keine vollständige und gleichmäßige Photooxidation der in dem Probeaufnahmegefäß befindlichen Urinprobe möglich ist. Es ist daher mit ungenauen Messungen der Phorphyrinwerte zu rechnen, da keine vollständige oder nahezu vollständige Photooxidation von beispielsweise vorhandenen Porphyrinogenen (die nicht fluoreszierende, nicht oxidierte Form der Porphyrine) zu Porphyrinen erfolgt.

Die vorliegende Erfindung befasst sich daher mit der Bereitstellung einer Möglichkeit zur zuverlässigen Aufnahme und Fixierung von Probeaufnahmegefäßen, insbesondere solchen, die für die Analyse fließfähiger Medien bestimmt sind, um eine konsistente und reproduzierbare Messung optischer Eigenschaften zu gewährleisten. Ziel ist es, eine stabile und definierte Positionierung der Proben zu ermöglichen, um Messfehler zu minimieren und die Genauigkeit der Analyse zu erhöhen. Ein weiterer Aspekt betrifft die Schaffung einer Konfiguration, die die gezielte Einwirkung von Licht auf das fließfähige Medium innerhalb des Probeaufnahmegefäßes ermöglicht, um spezifische Reaktionen auszulösen oder optische Signale zu erzeugen, die anschließend detektiert und ausgewertet werden können. Es soll eine effiziente und kontrollierte Anregung der Probe erreicht werden, um eine empfindliche und präzise Messung relevanter Komponenten zu ermöglichen. Dabei sollen die verwendeten Haltevorrichtungen und Messvorrichtungen möglichst kompakt gestaltet werden.

Diese Aufgaben werden erfindungsgemäß durch eine Haltevorrichtung mit den im Patentanspruch 1 angeführten Merkmalen sowie eine Messvorrichtung mit den Merkmalen des Anspruchs 7 gelöst. Vorteilhafte Ausgestaltungen mit zweckmäßigen Weiterbildungen der Erfindung sind in den jeweiligen Unteransprüchen angegeben, wobei vorteilhafte Ausgestaltungen der Haltevorrichtung als vorteilhafte Ausgestaltungen der Messvorrichtung und umgekehrt anzusehen sind.

Ein erster Aspekt der Erfindung betrifft eine Haltevorrichtung zur Aufnahme und Halterung von mindestens einem Probeaufnahmegefäß, wobei das Probeaufnahmegefäß transparent und/oder transluzent ausgebildet ist und einen Aufnahmeraum für ein zu untersuchendes, fließfähiges Medium aufweist. Die Haltevorrichtung umfasst dabei einen Halteblock mit mindestens zwei, an einem Basiselement angeordneten und parallel zueinander verlaufenden Halteblockschenkeln, wobei ein Zwischenraum zwischen den Halteblockschenkeln als Einführöffnung für das Probeaufnahmegefäß ausgebildet ist. Des Weiteren umfasst die Haltevorrichtung mindestens eine Beleuchtungseinrichtung zur Photooxidation des zu untersuchenden Mediums, wobei die Beleuchtungseinrichtung an einer in den Zwischenraum zeigenden Oberfläche von mindestens einem der Halteblockschenkel direkt oder indirekt angeordnet ist. Schließlich ist ein dem Basiselement des Halteblocks gegenüberliegendes Ende des Zwischenraums als Lichteinlaßöffnung für einen Anregungslichtstrahl ausgebildet.

Mit anderen Worten betrifft die Erfindung eine Haltevorrichtung, die ein Probeaufnahmegefäß sicher aufnimmt und fixiert, wobei die transparente oder transluzente Beschaffenheit des Gefäßes die Untersuchung des darin befindlichen fließfähigen Mediums ermöglicht. Die Haltevorrichtung besteht aus einem Halteblock mit mindestens zwei parallel angeordneten Schenkeln, die einen Zwischenraum als Einführöffnung für das Gefäß bilden. Ein dem Basiselement des Halteblocks gegenüberliegendes Ende dieses Zwischenraums dient als Lichteinlaßöffnung.

Die Integration einer Beleuchtungseinrichtung an einer Oberfläche mindestens eines der Halteblockschenkel, die in den Zwischenraum zeigt, ermöglicht eine effektive Photooxidation des zu untersuchenden Mediums. Dies ist besonders vorteilhaft, da die Anordnung eine vollständige und gleichmäßige Photooxidation gewährleistet, was bei Messungen, wie beispielsweise der Porphyrinbestimmung in Urin, zu präziseren Ergebnissen führt. Die Vermeidung einer Photooxidation über einen kleinen Öffnungsquerschnitt eines Probeaufnahmegefäßes, wie in bekannten Vorrichtungen, minimiert Ungenauigkeiten, die durch unvollständige Umwandlung von beispielsweise Porphyrinogenen zu Porphyrinen entstehen könnten. Durch die direkte oder indirekte Anordnung der Beleuchtungseinrichtung wird sichergestellt, dass das Anregungslicht effizient genutzt und eine optimale Ausleuchtung des Probeaufnahmegefäßes erreicht wird. Die Kombination aus sicherer Gefäßaufnahme und effektiver Photooxidation verbessert somit die Messgenauigkeit und Zuverlässigkeit der Analyse des fließfähigen Mediums. Zudem kann die Haltevorrichtung kompakt und damit auch platzsparend ausgebildet werden. Unter den Begriffen "direkte oder indirekte Anordnung" wird verstanden, dass die Beleuchtungseinrichtung direkt an der Oberfläche des Halteblockschenkels oder über ein Zwischenelement an dem Halteblockschenkel angeordnet ist.

In weiterer vorteilhafter Ausgestaltung der erfindungsgemäßen Haltevorrichtung ist vorgesehen, dass die Beleuchtungseinrichtung mindestens eine LED, insbesondere eine Hochleistungs-LED, umfasst. Mit anderen Worten ist es vorgesehen, dass die zur Beleuchtung des Probeaufnahmegefäßes und des zu untersuchenden Mediums vorgesehene Beleuchtungseinrichtung mindestens eine Leuchtdiode, vorzugsweise eine Leuchtdiode mit hoher Lichtstromdichte, beinhaltet.

Die Verwendung mindestens einer LED als Beleuchtungseinrichtung ermöglicht eine kompakte Bauweise der Haltevorrichtung, da LEDs im Vergleich zu herkömmlichen Lichtquellen geringere Abmessungen aufweisen. Dies resultiert in einer Reduktion des Platzbedarfs und ermöglicht eine Integration der Beleuchtungseinrichtung in die Haltevorrichtung ohne die Funktionalität oder Zugänglichkeit des Probeaufnahmegefäßes zu beeinträchtigen. Die Verwendung einer Hochleistungs-LED verstärkt diesen Effekt, da diese eine bei geringer Baugröße eine hohe Lichtstromdichte liefert. Folglich kann die Beleuchtungseinrichtung effizienter und platzsparender in die Haltevorrichtung integriert werden, wodurch die Gesamtgröße und Komplexität der Messvorrichtung reduziert werden. Die Verwendung von LEDs ermöglicht zudem eine präzisere Steuerung der Lichtintensität und des Wellenlängenbereichs, was eine optimierte Anregung der im Probeaufnahmegefäß befindlichen Probe und somit eine genauere Messung der relevanten Komponenten gewährleistet.

Dabei ist die LED oder Hochleistungs-LED geeignet, Licht abzustrahlen, welches eine Photooxidation des in dem Probeaufnahmegefäß aufgenommenen und zu untersuchenden Mediums bewirkt. Mit anderen Worten ist es vorgesehen, dass die lichtemittierende Diode oder die Hochleistungs-Lichtemittierende Diode Licht emittiert, welches eine chemische Reaktion, nämlich die Oxidation, in dem innerhalb des Probeaufnahmegefäßes befindlichen Medium auslöst. Die Verwendung einer LED oder Hochleistungs-LED, die Licht zur Bewirkung einer Photooxidation emittiert, ermöglicht eine gezielte und kontrollierte chemische Veränderung des zu untersuchenden Mediums innerhalb des Probeaufnahmegefäßes. Hierdurch wird sichergestellt, dass die im Medium eventuell vorhandenen, photooxidationsbedürftigen Komponenten einer entsprechenden Reaktion unterzogen werden, wodurch die Genauigkeit und Verlässlichkeit der nachfolgenden Messungen verbessert wird. Die direkte Anregung der Photooxidation durch die Lichtemission der LED oder Hochleistungs-LED vermeidet zudem die Notwendigkeit externer Lichtleiter oder anderer komplexer optischer Systeme zur Lichtübertragung, was zu einer Vereinfachung der Bauweise der Haltevorrichtung und der Messvorrichtung insgesamt führt. Die gezielte Photooxidation ermöglicht die Umwandlung von nicht messbaren Vorstufen in messbare Substanzen, was die analytische Leistungsfähigkeit der Messvorrichtung steigert.

Zudem kann die LED oder Hochleistungs-LED geeignet sein, Licht in einem Wellenlängenbereich von 450 nm bis 500 nm, insbesondere von 470 nm bis 480 nm, abzugeben. Mit anderen Worten ist es vorgesehen, dass die lichtemittierende Diode oder die Hochleistungs-Lichtemittierende Diode so konfiguriert ist, dass sie elektromagnetische Strahlung mit einer Wellenlänge zwischen 450 Nanometern und 500 Nanometern, vorzugsweise zwischen 470 Nanometern und 480 Nanometern, emittiert. Die Konfiguration der Lichtquelle, insbesondere der LED oder Hochleistungs-LED, zur Abgabe von Licht in dem genannten Wellenlängenbereich ermöglicht eine optimierte Oxidation von beispielsweise Porphyrogenen zu Porphyrinen. Dadurch wird sichergestellt, dass sich die Signalstärke der zu detektierenden Porphyrine erhöht, was zu einer verbesserten Messgenauigkeit führt. Zugleich wird der Wellenlängenbereich so gewählt, dass möglichst kein Ausbleichen der zu messenden Komponente (insbesondere der Porphyrine) stattfindet. Das heißt, die zu messende Komponente soll nicht oder nur schwach durch das Licht der LED angeregt werden. Dies steht im Gegensatz zum Licht der Laserdiode aus der Messeinheit, die gerade so gewählt ist, dass die zu messende Komponente möglichst stark zur Fluoreszenz angeregt wird.

In einer vorteilhaften Ausgestaltung der Erfindung ist vorgesehen, dass die Haltevorrichtung mindestens eine Transmissionsmessvorrichtung zur Messung einer Transmission des auf das Medium gerichteten Anregungslichtstrahls mindestens einer Anregungslichtquelle aufweist, wobei die Transmissionsmessvorrichtung an einer in den Zwischenraum zeigenden Oberfläche des Basiselements des Halteblocks direkt oder indirekt angeordnet ist. Mit anderen Worten ist es vorgesehen, dass die Haltevorrichtung eine Transmissionsmessvorrichtung umfasst, die die Durchlässigkeit des auf das fließfähige Medium gerichteten Anregungslichts misst, wobei diese Messvorrichtung an der Oberfläche des Basiselements des Halteblocks positioniert ist, die zum Zwischenraum zwischen den Halteblockschenkeln zeigt.

Durch die Anordnung der Transmissionsmessvorrichtung an der in den Zwischenraum zeigenden Oberfläche des Basiselements ist eine präzise Messung der Transmission des Anregungslichts durch das fließfähige Medium möglich, ohne dass die Messvorrichtung selbst den Lichtweg des Anregungslichts beeinträchtigt oder das Messergebnis verfälscht. Die direkte oder indirekte Anordnung der Transmissionsmessvorrichtung ermöglicht eine flexible Integration in die Haltevorrichtung, wobei eine optimale Positionierung zur Erfassung des transmittierten Lichts gewährleistet wird. Dies führt zu einer erhöhten Genauigkeit der Messung und somit zu zuverlässigeren Ergebnissen bei der quantitativen Bestimmung der in dem fließfähigen Medium enthaltenen Komponenten. Die räumliche Nähe der Transmissionsmessvorrichtung zum Probeaufnahmegefäß minimiert zudem Streulichteffekte und verbessert die Signal-Rausch-Verhältnisse, was zu einer weiteren Steigerung der Messgenauigkeit beiträgt. Dies bedeutet, dass durch die Messung der Transmission des Anregungslichtstrahls bei der Anregungswellenlänge eine Korrektur der gemessenen beziehungsweise detektierten optischen Signale erfolgen kann. Dies ist beispielweise bei üblicherweise unterschiedlich optisch dichten Medien, wie zum Beispiel Urin, von großem Vorteil. Die Transmissionsmessvorrichtung kann dabei mindestens eine Photodiode aufweisen. Unter den Begriffen "direkte oder indirekte Anordnung" wird hierbei verstanden, dass die Transmissionsmessvorrichtung direkt an der Oberfläche des Basiselements oder über ein Zwischenelement an dem Basiselement angeordnet ist.

Gemäß einer weiteren vorteilhaften Ausgestaltung der Haltevorrichtung sind die in den Zwischenraum gerichteten Flächen der Halteblockschenkel zumindest teilweise einem Außenumfang des aufzunehmenden Probeaufnahmegefäßes angepasst. Damit ist eine sichere und richtige Halterung des Probeaufnahmegefäßes gewährleistet. Zudem besteht die Möglichkeit, dass die die in den Zwischenraum gerichteten Flächen der Halteblockschenkel Führungsschienen zur Führung und Aufnahme des aufzunehmenden Probeaufnahmegefäßes aufweisen. Mit anderen Worten ist es vorgesehen, dass die den Hohlraum zwischen den Halteblockschenkeln begrenzenden Flächen mit strukturellen Elementen ausgebildet sind, die eine definierte Führung und Aufnahme des Probeaufnahmegefäßes ermöglichen. Durch die Integration von Führungsschienen in die der Halteblockschenkel zugewandten Flächen wird eine präzise Positionierung des Probeaufnahmegefäßes innerhalb der Haltevorrichtung gewährleistet. Dies minimiert jegliche laterale Verschiebung oder Rotation des Gefäßes und ermöglicht eine reproduzierbare Anordnung für Messzwecke oder andere Anwendungen. Die definierte Führung trägt dazu bei, dass die optischen oder sensorischen Elemente, die zur Analyse des Inhalts des Probeaufnahmegefäßes eingesetzt werden, stets auf die korrekte Stelle innerhalb des Gefäßes ausgerichtet sind. Die strukturelle Integration der Führungsschienen in die Halteblockschenkel gewährleistet zudem eine stabile und zuverlässige Fixierung des Probeaufnahmegefäßes, wodurch Vibrationen oder Störungen während der Messung oder Analyse ausgeschlossen werden. Die daraus resultierende verbesserte Stabilität und Präzision der Gefäßpositionierung trägt somit unmittelbar zu einer Erhöhung der Messgenauigkeit und -zuverlässigkeit bei.

In einer vorteilhaften Ausgestaltung der erfindungsgemäßen Haltevorrichtung ist vorgesehen, dass der Halteblock ganz oder teilweise aus einem wärmeaufnehmenden Material, insbesondere Aluminium oder Edelstahl, besteht. Mit anderen Worten ist es vorgesehen, dass der Halteblock entweder vollständig oder in Teilen aus einem Material gefertigt ist, welches Wärme leitet und somit Wärme aufnehmen kann, wobei Aluminium oder Edelstahl als bevorzugte Materialien in Frage kommen. Durch die Verwendung eines massiven, wärmeaufnehmenden Materials für den Halteblock wird eine effiziente Wärmepufferwirkung und Wärmeableitung von dem Probeaufnahmegefäß ermöglicht. Insbesondere bei der Photooxidation durch Hochleistungs-LEDs des in dem Probeaufnahmegefäß befindlichen fließfähigen Mediums, wie beispielsweise der Urinprobe, wird Wärme freigesetzt. Da das wärmeaufnehmende Material Wärme ableitet, puffert, und letztendlich an die Luft abgibt, wird eine Temperaturerhöhung des Probeaufnahmegefäßes und des darin befindlichen Mediums minimiert. Dies trägt dazu bei, die Messgenauigkeit zu verbessern, da die chemischen und physikalischen Eigenschaften des fließfähigen Mediums, welche für die Messung relevant sind, durch eine konstante Temperatur stabil gehalten werden. Folglich ermöglicht die Verwendung eines wärmeaufnehmenden Materials eine präzisere und zuverlässigere Bestimmung der in dem fließfähigen Medium vorkommenden fluoreszierenden, lichtabsorbierenden und/oder lichtrückstreuenden Komponente.

**In** einer vorteilhaften Ausgestaltung der erfindungsgemäßen Haltevorrichtung ist vorgesehen, dass die Beleuchtungseinrichtung auf einer Platine angeordnet ist, wobei ein Trägermaterial der Platine aus Aluminium oder Kupfer besteht. Die Anordnung der Beleuchtungseinrichtung auf einer Platine ermöglicht eine präzise und stabile Positionierung der Lichtquellen relativ zu dem Probeaufnahmegefäß, was zu einer verbesserten Lichtausbeute und einer gleichmäßigeren Beleuchtung der Probe führt. Die Verwendung von Aluminium oder Kupfer als Trägermaterial der Platine bietet ausgezeichnete thermische Eigenschaften, da beide Metalle eine hohe Wärmeleitfähigkeit besitzen. Dies ist von besonderer Bedeutung, da die Beleuchtungseinrichtung während des Betriebs Wärme erzeugt, die effektiv abgeführt werden muss, um eine Überhitzung der Komponenten zu vermeiden und deren Lebensdauer zu verlängern. Eine effiziente Wärmeableitung trägt somit zur Betriebssicherheit und Zuverlässigkeit der Haltevorrichtung bei und ermöglicht eine stabile und reproduzierbare Messung der fluoreszierenden, lichtabsorbierenden oder lichtrückstreuenden Komponenten in dem fließfähigen Medium. Die Verwendung von Metallen mit hoher Wärmeleitfähigkeit gewährleistet, dass die Wärme effizient von den Beleuchtungselementen abgeleitet wird, wodurch eine konstante Betriebstemperatur und eine langfristige Stabilität der Haltevorrichtung gewährleistet werden. Zudem besteht die Möglichkeit, dass die Platine an der in den Zwischenraum zeigenden Oberfläche von mindestens einem der Halteblockschenkel befestigt ist. Die Befestigung der Platine an der in den Zwischenraum zeigenden Oberfläche eines Halteblockschenkels ermöglicht eine kompakte Bauweise der Haltevorrichtung, da die Platine nicht zusätzlich fixiert werden muss und somit weniger Bauraum benötigt. Dies führt zu einer Reduzierung der Gesamtgröße der Haltevorrichtung und ermöglicht eine Integration in Messvorrichtungen mit begrenztem Platzangebot. Durch die Integration der Platine in die Struktur der Haltevorrichtung wird zudem die Stabilität und mechanische Festigkeit der gesamten Anordnung verbessert. Die Platine wird durch den Halteblockschenkel geschützt und kann somit weniger stark durch äußere Einflüsse beeinträchtigt werden.

In einer weiteren vorteilhaften Ausgestaltung der erfindungsgemäßen Haltevorrichtung ist an einer Außenfläche des Basiselementes mindestens ein Griffelement direkt oder indirekt angeordnet ist. Damit vereinfacht sich vorteilhafterweise die Handhabbarkeit der Haltevorrichtung.

Ein zweiter Aspekt der vorliegenden Erfindung betrifft eine Messvorrichtung zum quantitativen Bestimmen mindestens einer in einem fließfähigen Medium vorkommenden fluoreszierenden, lichtabsorbierenden und/oder lichtrückstreuenden Komponente umfassend ein Gehäuse, mindestens eine Haltevorrichtung gemäß dem ersten Aspekt der Erfindung, mindestens eine Einführöffnung in dem Gehäuse zur Aufnahme der Haltevorrichtung, mindestens eine Lichtquelle zur Erzeugung mindestens eines Anregungslichtstrahls mit mindestens einer vorbestimmten Wellenlänge, wobei der Anregungslichtstrahl über ein optisches System zu mindestens einem im Bereich der Einführöffnung angeordneten Lichtauslaß und der damit lichtleitend verbundenen Lichteinlaßöffnung der Haltevorrichtung leitbar ist, mindestens eine Steuereinrichtung zur Steuerung einer Belichtungszeit und/oder einem Belichtungsintervall des Anregungslichtstrahls und/oder einer Belichtungszeit und/oder einem Belichtungsintervall des von der Beleuchtungseinrichtung erzeugten Lichtstrahls, sowie mindestens eine Auswerteeinrichtung zum Empfang und quantitativen Auswertung der durch den Anregungslichtstrahl in dem zu analysierenden Medium erzeugten Absorption, Rückstreuung und/oder Fluoreszenz.

Mit anderen Worten betrifft die Erfindung eine Messvorrichtung, die dazu dient, die Konzentration von Substanzen in fließfähigen Medien präzise zu bestimmen, indem die Absorption, Rückstreuung oder Fluoreszenz dieser Substanzen gemessen wird. Die Vorrichtung beinhaltet ein Gehäuse, eine Haltevorrichtung für das Probengefäß, eine Einführöffnung für die Haltevorrichtung, eine Lichtquelle zur Erzeugung des Anregungslichts, ein optisches System zur Lichtleitung, eine Steuereinrichtung zur Regelung der Beleuchtung und eine Auswerteeinrichtung zur quantitativen Auswertung der Messsignale. Die Integration einer Haltevorrichtung in die Messvorrichtung ermöglicht eine präzise und reproduzierbare Positionierung des Probeaufnahmegefäßes relativ zur Lichtquelle des Anregungslichtstrahls. Die Einführöffnung in das Gehäuse dient dazu, die Haltevorrichtung sicher, einfach und definiert in die Messvorrichtung einzuführen. Die Lichtquelle erzeugt den benötigten Anregungslichtstrahl mit einer spezifischen Wellenlänge, der über das optische System zum Probeaufnahmegefäß geleitet wird. Die Steuereinrichtung ermöglicht die präzise Kontrolle der Belichtungszeit und des Belichtungsintervalls des Anregungslichtstrahls, was zu genaueren und zuverlässigeren Messergebnissen führt. Die Auswerteeinrichtung empfängt die durch den Anregungslichtstrahl im Probenmedium erzeugten Signale, wie Absorption, Rückstreuung oder Fluoreszenz, und wertet diese quantitativ aus, um die Konzentration der analysierten Komponente zu bestimmen. Dies ermöglicht eine genaue und effiziente Analyse fließfähiger Medien. Mittels der Beleuchtungseinrichtung erfolgt eine vordefinierte Photooxidation von im Medium eventuell vorhandenen, photooxidationsbedürftigen Komponenten, wodurch die Genauigkeit und Verlässlichkeit der nachfolgenden Messungen verbessert wird. Insgesamt ist zudem ein kompakter Aufbau der erfindungsgemäßen Messvorrichtung gewährleistet. Zudem führt die Kombination dieser Merkmale zu einer robusten und zuverlässigen Messvorrichtung, die präzise quantitative Ergebnisse liefert und somit für eine Vielzahl von Anwendungen in den Bereichen Chemie, Biologie, Medizin und Umweltanalytik geeignet ist. Im Zusammenhang mit der Detektion von Porphyrinen im Urin ermöglicht diese Vorrichtung eine genaue und zuverlässige Bestimmung der Porphyrinkonzentration, was für die Diagnose von Porphyrien und anderer Erkrankungen von entscheidender Bedeutung ist. Die Möglichkeit, die Messparameter anzupassen, trägt zur Reduzierung von Störeffekten und zur Verbesserung der Messgenauigkeit bei.

In einer vorteilhaften Ausgestaltung der Erfindung ist daher vorgesehen, dass das Medium Urin ist und/oder die zu analysierende Komponente aus der Gruppe der Porphyrine gewählt ist. Mit anderen Worten ist es vorgesehen, dass die Messvorrichtung zur quantitativen Bestimmung mindestens einer in einem fließfähigen Medium vorkommenden Komponente konzipiert ist, wobei dieses fließfähige Medium aus Urin besteht und/oder die zu analysierende Komponente einer aus der Gruppe der Porphyrine stammt. Die Verwendung von Urin als zu analysierendes Medium ermöglicht eine spezifische Anpassung der Messvorrichtung an die besonderen Eigenschaften dieser Körperflüssigkeit, was die Genauigkeit und Zuverlässigkeit der Messungen verbessern kann. Die Auswahl der Porphyrine als zu analysierende Komponente erlaubt die gezielte Detektion und Quantifizierung dieser Substanzen in Urin, was insbesondere im Bereich der medizinischen Diagnostik von Bedeutung ist. Die Konzentration von Porphyrinen in Urin kann als Indikator für verschiedene Erkrankungen dienen, und eine präzise Messung dieser Konzentration ist daher von entscheidender Bedeutung für eine korrekte Diagnose.

In einer vorteilhaften Ausgestaltung der erfindungsgemäßen Messvorrichtung ist vorgesehen, dass die Messvorrichtung mindestens eine Bedien- und/oder Anzeigevorrichtung umfasst. Mit anderen Worten ist es vorgesehen, dass die Messvorrichtung über eine oder mehrere Einrichtungen verfügt, die eine Interaktion mit dem Benutzer ermöglichen und/oder Ergebnisse der Messung visuell darstellen. Die Integration einer Bedien- und/oder Anzeigevorrichtung in die Messvorrichtung ermöglicht eine benutzerfreundlichere Handhabung der Messvorrichtung und erleichtert die Durchführung der Messung. Durch die Bedien- und/oder Anzeigevorrichtung kann der Benutzer die Messvorrichtung steuern und die Ergebnisse der Messung unmittelbar ablesen. Dies trägt zu einer effizienteren und präziseren Durchführung der Messung bei. Die unmittelbare Ablesbarkeit der Messergebnisse, die durch die Anzeigevorrichtung ermöglicht wird, vermeidet zudem potenzielle Fehlerquellen, die bei der manuellen Übertragung von Messwerten entstehen könnten. Somit verbessert die vorgesehene Ausstattung die Bedienbarkeit und erhöht die Zuverlässigkeit der Messvorrichtung.

In einer weiteren vorteilhaften Ausgestaltung der erfindungsgemäßen Messvorrichtung ist vorgesehen, dass ein Detektionslichtstrahl über die Lichteinlaßöffnung der Haltevorrichtung und den Lichtauslaß des Gehäuses zu der Auswerteeinrichtung geleitet wird. Mit anderen Worten ist es vorgesehen, dass der Detektionslichtstrahl, der zur Erfassung der Informationen dient, die von der Probenemission erzeugt wurden, den Weg über die Lichteinlaßöffnung der Haltevorrichtung und den Lichtauslaß des Gehäuses nimmt, um zur Auswerteeinrichtung zu gelangen. Durch die beschriebene Lichtführung wird eine präzise und definierte Streckenführung des Detektionslichtstrahls erreicht. Dies trägt dazu bei, Signalverluste durch Streuung oder Absorption des Lichts auf seinem Weg zur Auswerteeinrichtung zu minimieren, wodurch die Messgenauigkeit verbessert wird. Darüber hinaus ermöglicht die gezielte Führung des Lichtstrahls eine optimierte Anordnung der optischen Komponenten, was zu einer kompakteren Bauweise der Messvorrichtung beitragen kann. Die Integration der Lichtführung in die Struktur der Haltevorrichtung und des Gehäuses gewährleistet eine stabile und zuverlässige Führung des Lichtstrahls, wodurch die Robustheit und Langzeitstabilität der Messvorrichtung erhöht wird. Die beschriebene Lichtführung ermöglicht somit eine effiziente Erfassung der von der Probe emittierten Signale und trägt zur Steigerung der Genauigkeit und Zuverlässigkeit der Messvorrichtung bei.

In einer vorteilhaften Ausgestaltung der Erfindung ist vorgesehen, dass die Messvorrichtung mindestens eine Anregungslichtquelle, insbesondere Laserlichtquelle, umfasst, wobei die Anregungslichtquelle einen ersten Anregungslichtstrahl innerhalb eines Wellenlängenbereichs von 385 nm bis 410 nm, insbesondere mit einer Zentralwellenlänge von 400 nm bis 401 nm, erzeugt. Mit anderen Worten ist es vorgesehen, dass die Messvorrichtung eine Lichtquelle beinhaltet, welche einen Lichtstrahl mit einer Zentralwellenlänge zwischen 385 und 410 Nanometern emittiert, wobei ein besonders bevorzugter Wellenlängenbereich zwischen 400 und 401 Nanometern liegt. Die Verwendung einer Anregungslichtquelle, welche einen ersten Anregungslichtstrahl innerhalb des spezifizierten Wellenlängenbereichs erzeugt, ermöglicht eine gezielte Anregung von fluoreszierenden Komponenten innerhalb des fließfähigen Mediums. Die präzise Wahl des Wellenlängenbereichs unterstützt eine effiziente Anregung der zu messenden Komponenten und trägt somit zur Optimierung des Messsignals bei. Durch die Konzentration der Anregungsenergie auf einen eng begrenzten Wellenlängenbereich wird die Wahrscheinlichkeit einer unerwünschten Anregung von Störkomponenten innerhalb des fließfähigen Mediums reduziert, was die Genauigkeit und Zuverlässigkeit der Messung insgesamt verbessert. Die Verwendung einer derart spezifizierten Anregungslichtquelle trägt somit zu einer präziseren quantitativen Bestimmung der in dem fließfähigen Medium vorhandenen fluoreszierenden Komponenten, insbesondere von Porphyrinen im Urin, bei. Es besteht aber auch die Möglichkeit, dass die Messvorrichtung eine zweite Anregungslichtquelle umfasst, insbesondere eine weitere Laserlichtquelle, wobei die zweite Anregungslichtquelle einen zweiten Anregungslichtstrahl mit einer von dem Wellenlängenbereich des ersten Anregungslichtstrahls unterschiedlichen Wellenlängenbereich erzeugt. Mit anderen Worten ist es vorgesehen, dass die Messvorrichtung zusätzlich zu einer ersten Anregungslichtquelle eine zweite Anregungslichtquelle aufweist, die Licht mit einer anderen Wellenlänge als die erste Anregungslichtquelle emittiert. Die Bereitstellung einer zweiten Anregungslichtquelle, die einen Anregungslichtstrahl mit einem von dem der ersten Anregungslichtquelle unterschiedlichen Wellenlängenbereich erzeugt, ermöglicht eine gezielte und selektive Anregung verschiedener Komponenten innerhalb des fließfähigen Mediums. Dadurch wird die Möglichkeit geschaffen, unterschiedliche fluoreszierende, lichtabsorbierende oder lichtrückstreuende Komponenten im Medium zu identifizieren und quantitativ zu bestimmen, da jede Komponente potenziell bei einer spezifischen Wellenlänge optimal angeregt werden kann. Dies führt zu einer erhöhten Messgenauigkeit und ermöglicht eine differenzierte Analyse des fließfähigen Mediums, da die Signale der einzelnen Komponenten besser voneinander getrennt und quantifiziert werden können. Insbesondere bei komplexen Medien mit mehreren Komponenten ermöglicht die Verwendung unterschiedlicher Wellenlängenbereiche eine präzisere Bestimmung der Konzentration jeder einzelnen Komponente.

In einer weiteren vorteilhaften Ausgestaltung der erfindungsgemäßen Messvorrichtung ist vorgesehen, dass die Transmissionsmessvorrichtung der Haltevorrichtung mit der der Auswerteeinrichtung datenleitend verbunden ist. Mit anderen Worten ist es vorgesehen, dass eine Datenverbindung zwischen der Transmissionsmessvorrichtung und der Auswerteeinrichtung besteht, die die Übertragung von Messdaten ermöglicht. Durch die datenleitende Verbindung zwischen der Transmissionsmessvorrichtung und der Auswerteeinrichtung wird ermöglicht, dass die von der Transmissionsmessvorrichtung erfassten Messdaten unmittelbar und automatisiert an die Auswerteeinrichtung übermittelt werden können. Dies ermöglicht eine zeitnahe und effiziente Verarbeitung der Messdaten durch die Auswerteeinrichtung, was die Geschwindigkeit und Genauigkeit der Bestimmung der in dem fließfähigen Medium vorkommenden fluoreszierenden, lichtabsorbierenden und/oder lichtrückstreuenden Komponente verbessert. Die datenleitende Verbindung vermeidet zudem manuelle Eingriffe bei der Datenübertragung, wodurch die Fehlerwahrscheinlichkeit reduziert und die Reproduzierbarkeit der Messung erhöht wird. Ferner ermöglicht die direkte Datenübertragung eine Integration der Messdaten in komplexe Auswertungsalgorithmen und -routinen, die in der Auswerteeinrichtung implementiert sind, was eine detailliertere und umfassendere Analyse des fließfähigen Mediums ermöglicht.

Gemäß weiteren vorteilhaften Ausgestaltungen der erfindungsgemäßen Messvorrichtung ist vorgesehen, dass die Steuereinrichtung die Beleuchtungseinrichtung derart steuert, dass mit dem erzeugten Lichtstrahl eine Belichtung und Photooxidation des zu untersuchenden Mediums vor einer Bestrahlung mit dem Anregungslichtstrahl, oder eine Belichtung und Photooxidation des zu untersuchenden Mediums nach einer Bestrahlung mit dem Anregungslichtstrahl, oder eine Belichtung und Photooxidation des zu untersuchenden Mediums vor und nach einer Bestrahlung mit dem Anregungslichtstrahl, erfolgt. Mit anderen Worten ist es vorgesehen, dass die Steuereinrichtung die Beleuchtungseinrichtung so ansteuert, dass das zu untersuchende Medium entweder vor, nach oder sowohl vor als auch nach der Bestrahlung mit dem Anregungslichtstrahl einer Belichtung und Photooxidation unterzogen wird. Die Steuerung der Beleuchtungseinrichtung zur Durchführung einer Belichtung und Photooxidation des zu untersuchenden Mediums vor einer Bestrahlung mit dem Anregungslichtstrahl ermöglicht die Vorbereitung des Mediums auf die nachfolgende Messung. Durch die Photooxidation können beispielsweise Porphyrinogene in Porphyrine umgewandelt werden, was die Genauigkeit der Messung von Porphyrinen erhöht. Ebenso ermöglicht die Durchführung einer Messung mit dem Anregungslichtstrahl vor der Belichtung zur Photooxidation die ursprünglich in der Probe vorhandene Porphyrine, ohne Umwandlung der Porphyrinogene, zu messen. Die Durchführung einer Belichtung und Photooxidation nach einer Bestrahlung mit dem Anregungslichtstrahl ermöglicht zudem eine eventuelle Nachbehandlung des Mediums und kann somit die Messgenauigkeit verbessern. Die Möglichkeit, die Belichtung und Photooxidation sowohl vor als auch nach der Bestrahlung mit dem Anregungslichtstrahl durchzuführen, eröffnet die Flexibilität, die optimale Vorgehensweise für das jeweilige zu untersuchende Medium und die gewünschte Messung zu wählen, insbesondere erlaubt dies auch den Zugewinn an Porphyrinen durch die Photooxidation von Porphyrinogenen zu bestimmen und so auf die ursprünglich vorhandene Porphyrinogenkonzentration zu schließen. Die vorgenannte Steuerung der Beleuchtungseinrichtung trägt somit insgesamt zur Verbesserung der Messgenauigkeit und -zuverlässigkeit bei.

Dabei kann die Steuereinrichtung die Beleuchtungseinrichtung und die Anregungslichtquelle derart steuern, dass die Anzahl der Mess-/Photooxidationszyklen in dem zu untersuchenden Medium zwischen 1 und 40, insbesondere zwischen 15 und 25, liegt. Es hat sich herausgestellt, dass eine derartige Anzahl an Mess-/Photooxidationszyklen zu verbesserten Messergebnissen gegenüber bekannten Messmethoden führt.

In weiteren vorteilhaften Ausgestaltungen der erfindungsgemäßen Messvorrichtung ist vorgesehen, dass die Steuereinrichtung die Beleuchtungseinrichtung derart steuert, dass eine Belichtungszeit für die Photooxidation zwischen 50 ms und 500 ms, insbesondere 150 ms, beträgt. Mit anderen Worten ist es vorgesehen, dass die Steuerung der Beleuchtungseinrichtung so erfolgt, dass die Dauer der Bestrahlung zur Photooxidation im Bereich von 50 Millisekunden bis 500 Millisekunden liegt, wobei eine bevorzugte Belichtungszeit von 150 Millisekunden besteht. Mit anderen Worten kann die Belichtungszeit 50 ms, 60 ms, 70 ms, 80 ms, 90 ms, 100 ms, 110 ms, 120 ms, 130 ms, 140 ms, 150 ms, 160 ms, 170 ms, 180 ms, 190 ms, 200 ms, 210 ms, 220 ms, 230 ms, 240 ms, 250 ms, 260 ms, 270 ms, 280 ms, 290 ms, 300 ms, 310 ms, 320 ms, 330 ms, 340 ms, 350 ms, 360 ms, 370 ms, 380 ms, 390 ms, 400 ms, 410 ms, 420 ms, 430 ms, 440 ms, 450 ms, 460 ms, 470 ms, 480 ms, 490 ms, 500 ms betragen. Weitere Zwischenwerte sind möglich. Die Festlegung einer Belichtungszeit für die Photooxidation zwischen 50 ms und 500 ms ermöglicht beispielsweise eine optimierte Umwandlung von nicht-fluoreszierenden Vorläuferstoffen in fluoreszierende Substanzen, wodurch eine höhere Empfindlichkeit und Genauigkeit der Messung ermöglicht wird. Durch die präzise Steuerung der Belichtungszeit wird sichergestellt, dass die Photooxidation effizient abläuft, ohne dabei die zu untersuchende Probe unnötig zu belasten oder photochemische Nebenreaktionen zu induzieren. Die bevorzugte Belichtungszeit von 150 ms stellt eine ausgewogene Balance zwischen der Effizienz der Photooxidation und der Minimierung von Störeffekten dar, wodurch die Reproduzierbarkeit und Zuverlässigkeit der Messergebnisse verbessert werden. Die vorgenannte Steuerung der Belichtungszeit trägt somit dazu bei, die Qualität und Aussagekraft der durchgeführten Messungen zu erhöhen.

Des Weiteren besteht die Möglichkeit, dass die Steuereinrichtung die Anregungslichtquelle derart steuert, dass eine Bestrahlung des zu untersuchenden Mediums mit dem Anregungslichtstrahl zwischen 5 ms und 50 ms, insbesondere für 15 ms, erfolgt. Auch diese Belichtungszeiten liefern verbesserte Messresultate, insbesondere bei der Messung von Porphyrinkonzentrationen in einer Urinprobe.

In einer weiteren vorteilhaften Ausgestaltung der erfindungsgemäßen Messvorrichtung umfasst die Beleuchtungseinrichtung zwischen 10 und 25 LEDs, wobei eine Lichtleistung pro LED zwischen 1 W und 3,5 W, insbesondere 3 W, beträgt. Damit sind deutliche und vollständige beziehungsweise nahezu vollständige Oxidationsreaktionen in dem zu untersuchen den Medium gewährleistet.

In einer weiteren vorteilhaften Ausgestaltung der erfindungsgemäßen Messvorrichtung ist die Steuereinrichtung und/oder die Auswerteeinheit in der Bedien- und Anzeigevorrichtung integriert ausgebildet. Die Bedien- und Anzeigevorrichtung kann dabei beispielsweise ein Tablet-Computer oder ein Desktop-Computer sein. Zudem besteht aber auch die Möglichkeit, dass die Steuereinrichtung und/oder die Auswerteeinheit von der Bedien- und Anzeigevorrichtung getrennt ausgebildet sind. So kann beispielsweise die Auswerteeinrichtung auch in einem Spektrometer der Messvorrichtung angeordnet sein. Es besteht zudem die Möglichkeit, dass Auswertung der Daten auf einem von der Messvorrichtung getrennt ausgebildeten Datenserver erfolgt. Hierfür werden die benötigten Messdaten kabelgebunden oder über entsprechende Funknetze zu dem Datenserver übertragen. Entsprechend werden die Messergebnisse wieder an die Bedien- und Anzeigevorrichtung übertragen. Insgesamt ergeben sich dadurch verschiedenste Möglichkeiten zur kompakten Ausgestaltung der erfindungsgemäßen Messvorrichtung.

In einer weiteren Ausgestaltung der erfindungsgemäßen Messvorrichtung leitet das optische System den Anregungslichtstrahl der Anregungslichtquelle sowie einen von dem Medium rückgestreuten und/oder emittierten Detektionslichtstrahl über Wegsamkeiten innerhalb der Messvorrichtung, des Gehäuses der Messvorrichung oder des optischen Systems. Damit kann vorteilhafterweise auf die Verwendung von Lichtleitern verzichtet werden. Dies führt zu einem einfacheren und damit auch kostengünstigeren Aufbau der erfindungsgemäßen Messvorrichtung.

Weitere Merkmale und deren Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Es zeigen:
- Fig. 1: eine schematische Darstellung einer erfindungsgemäßen Messvorrichtung;
- Fig. 2: eine schematische Detaildarstellung der erfindungsgemäßen Messvorrichtung gemäß Fig. 1;
- Fig. 3a und 3b: schematische Darstellungen einer erfindungsgemäßen Haltevorrichtung;
- Fig. 4: eine schematische Schnittdarstellung der erfindungsgemäßen Haltevorrichtung gemäß den Fig. 3a und 3b;
- Fig. 5: eine schematische Schnittdarstellung der erfindungsgemäßen Haltevorrichtung gemäß Fig. 4;
- Fig. 6: eine schematische Darstellung einer erfindungsgemäßen Messvorrichtung; und
- Fig. 7: ein Diagramm zur Darstellung der Effizienz der Anzahl von Photooxidationszyklen.

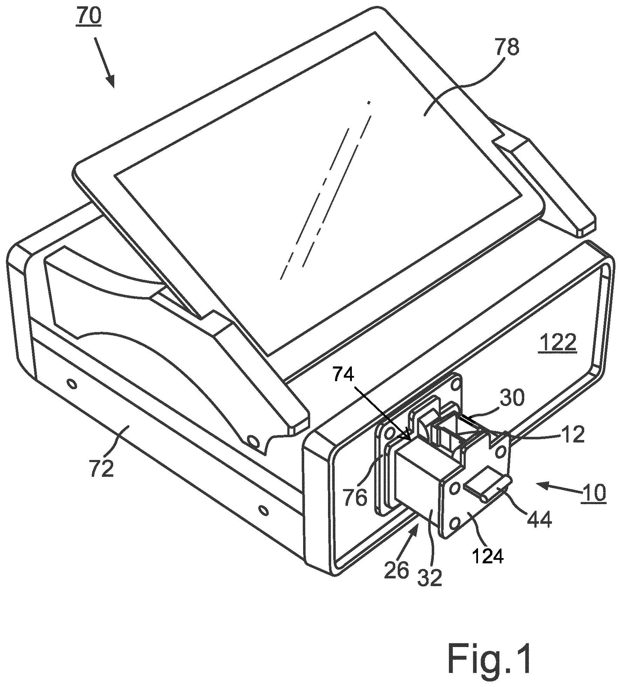

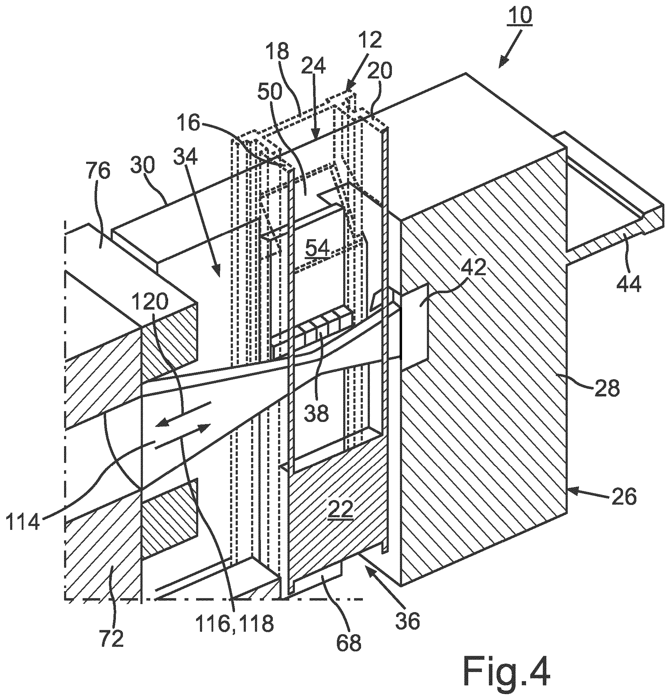

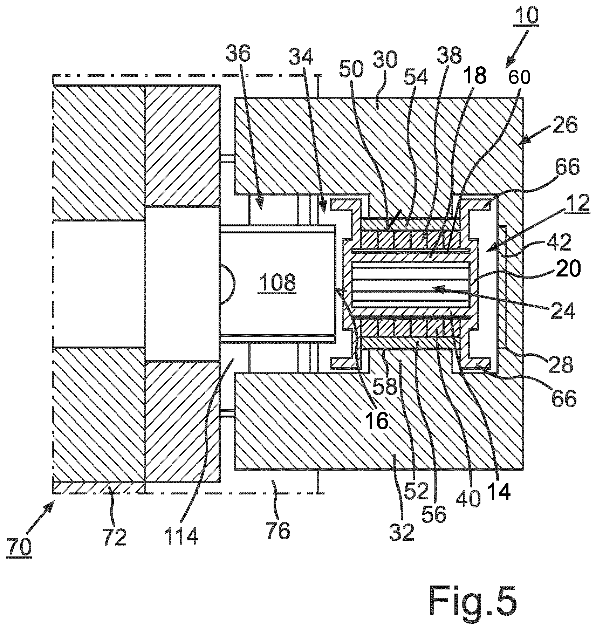

In den Figuren 1 bis 6 sind identische Merkmale mit gleichen Bezugszeichen versehen. Eine Haltevorrichtung 10 zur Aufnahme und Halterung von mindestens einem Probeaufnahmegefäß 12 sowie eine Messvorrichtung 70 zum quantitativen Bestimmen mindestens einer in einem fließfähigen Medium vorkommenden fluoreszierenden, lichtabsorbierenden und/oder lichtrückstreuenden Komponente wird im Folgenden in der Zusammenschau der Figuren 1 bis 6 beschrieben.

**Figur 1** zeigt eine schematische Darstellung einer Messvorrichtung 70. Die Messvorrichtung dient zum quantitativen Bestimmen mindestens einer in einem fließfähigen Medium vorkommenden, fluoreszierenden, lichtabsorbierenden und/oder lichtstreuenden Komponente. Insbesondere kann die dargestellte Messvorrichtung 70 zur Bestimmung von Porphyrinwerten im menschlichen Urin verwendet werden. Die Messvorrichtung 10 umfasst dabei ein Gehäuse 72 mit einer Einführöffnung 74 zur Aufnahme einer Haltevorrichtung 10. Die Haltevorrichtung 10 dient dabei zur Aufnahme und Halterung von einem Probeaufnahmegefäß 12, wobei das Probeaufnahmegefäß 12 das zu untersuchende Medium, hier Urin, beinhaltet. Die Haltevorrichtung 10 umfasst zudem eine Beleuchtungseinrichtung 38, 40 zur Photooxidation des zu untersuchenden Mediums und wird in den folgenden Figuren noch näher beschrieben.

Man erkennt, dass zur Ausbildung und Umrahmung der Einführöffnung 74 ein Rahmen 76 mit einer Kulisse an einer Frontplatte bzw. Vorderwand 122 des Gehäuses 72 befestigt ist. Die Ausnehmung im Rahmen 76 entspricht dabei ungefähr einem Außenumfang der Haltevorrichtung 10 in diesem Bereich. Damit wird einerseits gewährleistet, dass die Haltevorrichtung 10 immer in korrekter Richtung in das Gehäuse 72 der Messvorrichtung 70 eingeführt wird. Zudem verhindert der Rahmen 76, dass mögliches Streulicht über die Einführöffnung 74 in das Gehäuse 72 gelangen kann. Derartige Streulichteinflüsse würden das Messergebnis möglicherweise verfälschen.

Des Weiteren erkennt man, dass die Messvorrichtung 70 eine Bedien- und Anzeigevorrichtung 78 aufweist. In dem dargestellten Ausführungsbeispiel ist die Bedien- und Anzeigevorrichtung 78 ein Tablet-Computer.

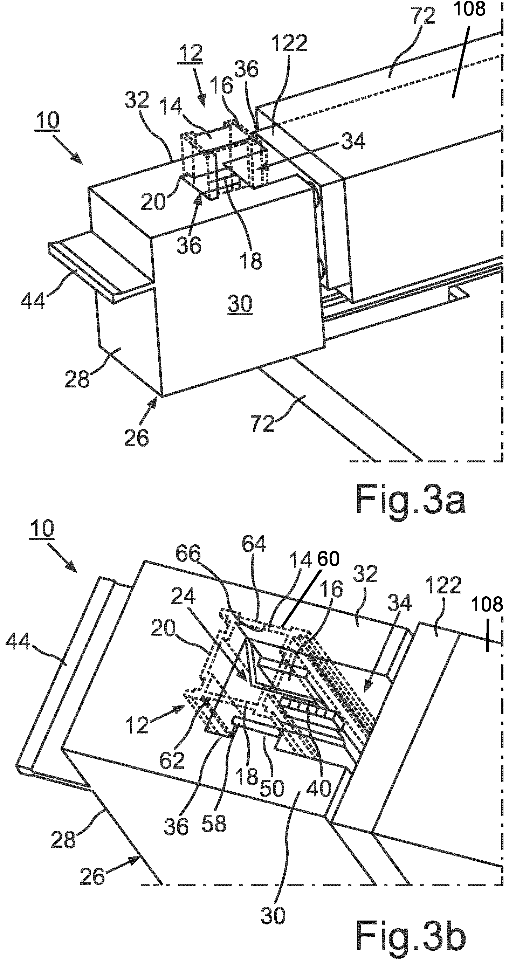

Des Weiteren erkennt man, dass die Haltevorrichtung 10 aus einem U-förmigen Halteblock 26 besteht. Ausgehend von einem Basisblock 28 (vergleiche auch Figuren 3a und 3b) sind zwei Halteblockschenkel 30, 32 angeordnet, die einen Aufnahmeraum 24 für das Probeaufnahmegefäß 12 ausbilden. Zudem ist an dem Basisblock 28 des Halteblocks 26 eine Griffplatte 124 befestigt, wobei an der Griffplatte 124 ein Griff 44 ausgebildet ist.

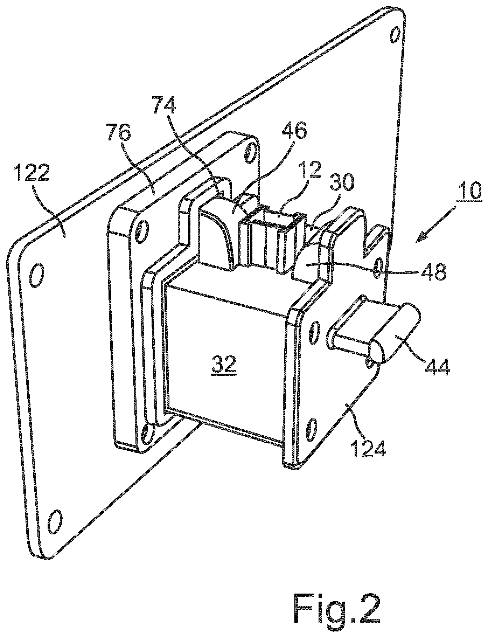

**Figur 2** zeigt eine schematische Detaildarstellung der Messvorrichtung 70 gemäß Figur 1. Dargestellt ist die Frontplatte 122 mit dem daran angeordneten Rahmen 76. Man erkennt wiederum, dass die Einführöffnung 74 im Rahmen 76 einem Außenumfang der Haltevorrichtung 10 in dem dem Basisblock 28 gegenüberliegenden Ende der Haltevorrichtung 10 entspricht. Zudem wird die U-förmige Ausgestaltung des Halteblocks 26 mit den beiden Halteblockschenkeln 30, 32 deutlich. Ein Zwischenraum zwischen den Halteblockschenkeln 30, 32 dient unter anderem als Einführungsöffnung 36 für das Probeaufnahmegefäß 12. Der Zwischenraum zwischen den Halteblockschenkeln 30, 32 ist zudem an seinem jeweiligen oberen Ende mit entsprechenden Abdunkelungskappen 46, 48 verschlossen. Die Einführöffnung 36 für das Probeaufnahmegefäß 12 liegt zwischen den Abdunkelungskappen 46, 48. Durch die Abdunkelungskappen 46, 48 wird zuverlässig verhindert, dass zusätzliches Streulicht aus der Umgebung über den Zwischenraum zwischen den Halteblockschenkeln 30, 32 in die Messvorrichtung 70 eindringen kann.

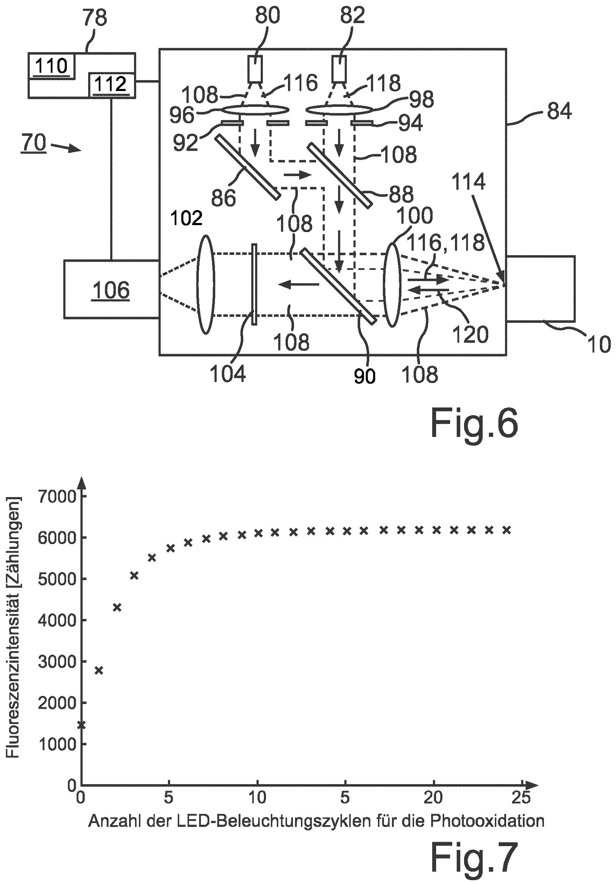

Bevor nunmehr im Einzelnen und detailliert auf die Ausgestaltung der Haltevorrichtung 10 eingegangen wird, wird im Folgenden der Aufbau der hier dargestellten Messvorrichtung 70 anhand der schematischen Darstellung gemäß **Figur 6** beschrieben. Man erkennt, dass die Messvorrichtung 70 in dem dargestellten Ausführungsbeispiel zwei Lichtquellen 80, 82 zur Erzeugung zweier Anregungslichtstrahlen 116, 118 mit jeweils einer vorbestimmten Wellenlänge umfasst. Bei den Anregungslichtquellen 80, 82 handelt es sich jeweils um eine Laserlichtquelle. Andere Lichtquellen, wie zum Beispiel LEDs sind ebenfalls denkbar. Zur Analyse von Komponenten aus der Gruppe der Porphyrine in dem Medium Urin erzeugt die erste Anregungslichtquelle 80 einen ersten Anregungslichtstrahl 116 innerhalb eines Wellenlängenbereichs von 385 nm bis 410 nm, insbesondere von 400 nm bis 401 nm. Die in dem Ausführungsbeispiel dargestellte zweite Anregungslichtquelle 82 erzeugt den zweiten Anregungslichtstrahl 118 mit einer von dem Wellenlängenbereich des ersten Anregungslichtstrahls 116 unterschiedlichen Wellenlänge. Damit können neben den genannten Porphyrinen auch andere in der Urinprobe enthaltenen Komponenten analysiert und quantitativ bestimmt werden. Es ist aber anzumerken, dass die zweite Lichtquelle 82 optional ist. Dies bedeutet, dass Messvorrichtungen 70 der hier beschriebenen Art auch lediglich eine erste Lichtquelle 80 umfassen können.

Des Weiteren erkennt man, dass die Anregungslichtstrahlen 116, 118 über ein optisches System 84 zu einem im Bereich der Einführöffnung 74 des Gehäuses 72 ausgebildeten Lichtauslass 114 und der damit lichtleitend verbundenen Lichteinlassöffnung 34 der Haltevorrichtung 10 geleitet werden. Der Strahlweg der Anregungslichtstrahlen 116, 118 erfolgt dabei über innerhalb des Gehäuses 72 der Messvorrichtung 70 ausgebildete Wegsamkeiten 108. Auf eine Strahlleitung über entsprechende Lichtleiterfasern kann verzichtet werden.

Das gemäß dem Ausführungsbeispiel schematisch dargestellte optische System 84 umfasst zudem im Strahlengang nach den Lichtquellen 80, 82 angeordnete optische Linsen 96, 98 sowie danach folgende Blenden 92, 94. Der erste Anregungslichtstrahl 116 wird nach dem Durchtritt durch die Blende 92 mittels eines optischen Spiegels 86 über eine entsprechende Wegsamkeit 108 in den Strahlengang bzw. die Wegsamkeit 108 des zweiten Anregungslichtstrahls 118 geleitet. Der Eintritt in den Strahlengang des zweiten Anregungslichtstrahls 118 erfolgt über einen optischen Strahlteiler 88. Nach dem optischen Strahlteiler 88 ist innerhalb der Wegsamkeit 108 ein weiterer optischer Strahlteiler 90 ausgebildet, der die Anregungslichtstrahlen 116, 118 über eine weitere optische Linse 100 zu dem Lichtauslass 114 und dem in der Haltevorrichtung 10 angeordneten Probeaufnahmegefäß 12 bzw. dem darin angeordneten und zu analysierenden Medium leitet. Eine durch die Anregungslichtstrahlen 116, 118 angeregte und von dem Medium emittierte Detektionslichtstrahlung 120 wird über den Lichtauslass 114 und den Strahlteiler 90 zu einem Spektrometer 106 geleitet. Im Strahlengang der Detektionslichtstrahlung 120 befinden sich vor dem Spektrometer 106 noch eine optische Filtereinrichtung 104 sowie eine weitere optische Linse 102. Die über die Detektionslichtstrahlung 120 an das Spektrometer 106 übertragenen Messdaten werden an eine in der Bedien- und Anzeigevorrichtung 78 ausgebildete Auswerteeinheit 112 über eine entsprechende Datenleitung geleitet. Die entsprechend ausgewerteten Messergebnisse können dann über die Bedien- und Anzeigevorrichtung 78 dargestellt werden. Des Weiteren ist anzumerken, dass das Spektrometer 106 auch als Auswerteeinrichtung zur Verarbeitung der empfangenen Messdaten und damit auch zur quantitativen Auswertung der durch die Anregungslichtstrahlen 116, 118 in dem zu analysierenden Medium erzeugten Absorption, Rückstreuung und Schrägstrich oder Fluoreszenz dienen kann.

Die Messvorrichtung 70 weist zudem eine Steuereinrichtung 110 auf, die zur Steuerung einer Belichtungszeit und/oder einem Belichtungsintervall der Anregungslichtstrahlen 116, 118 und/oder einer Belichtungszeit und/oder einem Belichtungsintervall des von der Beleuchtungseinrichtung 38, 40 der Haltevorrichtung 10 erzeugten Lichtstrahls, ausgebildet ist (vergleiche auch Figuren 3b bis 5). In dem dargestellten Ausführungsbeispiel ist die Steuereinrichtung 110 derart ausgebildet, dass sie die Beleuchtungseinrichtung 38, 40 derart ansteuert, dass mit dem erzeugten Lichtstrahl eine Belichtung und Photooxidation des zu untersuchenden Mediums vor und nach einer Bestrahlung mit dem ersten oder zweiten Anregungslichtstrahl 116, 118 erfolgt. Es ist aber auch denkbar, dass die Steuereinrichtung 110 die Beleuchtungseinrichtung 38, 40 derart steuert, dass mit dem erzeugten Lichtstrahl eine Belichtung und Photooxidation des zu untersuchenden Mediums nur vor oder nach einer Bestrahlung mit dem ersten oder zweiten Anregungslichtstrahl 116, 118 erfolgt. Die Steuereinrichtung 110 ist zudem derart ausgebildet, dass sie die Beleuchtungseinrichtung 38, 40 und die Anregungslichtquelle 80, 82 derart steuert, dass die Anzahl der Mess-Photooxidationszyklen des zu untersuchenden Medium zwischen 5 und 25 liegt. Die Steuereinrichtung 110 kann zudem die Beleuchtungseinrichtung 38, 40 derart steuern, dass eine Belichtungszeit für die Photooxidation zwischen 100 ms und 200 ms, insbesondere 150 ms, beträgt. Schließlich ist die Steuereinrichtung 110 derart ausgebildet, dass die Anregungslichtquellen 80, 82 derart angesteuert werden, dass eine Bestrahlung des zu untersuchenden Mediums mit den Anregungslichtstrahlen 116, 118 jeweils zwischen 10 ms und 25 ms, insbesondere für 15 ms, erfolgt. Die Steuereinrichtung 110 ist in dem dargestellten Ausführungsbeispiel ebenfalls in die Bedien- und Anzeigevorrichtung 78 integriert.

Des Weiteren erkennt man, dass auch der von dem Medium rückgestreute und/oder emittierte Detektionslichtstrahl 120 über Wegsamkeiten 108 innerhalb der Messvorrichtung 70 geleitet wird. Auch hier kann wiederum auf die Verwendung von separaten Lichtleitfasern oder Lichtleitfaserbündel verzichtet werden.

Die **Figuren 3a bis 5** zeigen die Haltevorrichtung 10 in detaillierteren Ansichten. Die Haltevorrichtung 10 wird im Folgenden in der Zusammenschau der Figuren 3a bis 5 beschrieben. 108Man erkennt, dass die Haltevorrichtung 10 zur Aufnahme und Halterung des Probeaufnahmegefäßes 12 dient. Das Probeaufnahmegefäß 12 ist dabei transparent ausgebildet. Zudem weist das Probeaufnahmegefäß 12 einen Aufnahmeraum 24 für das zu untersuchende fließfähige Medium auf. Man erkennt, dass der Aufnahmeraum 24 durch Seitenwände 14, 16, 18, 20 und eine Bodenwand 22 begrenzt wird.

Wie bereits dargelegt, umfasst die Haltevorrichtung 10 den Halteblock 26, der U-förmig ausgebildet ist. An den beiden parallel zueinander verlaufenden Halteblockschenkeln 30, 32 ist dabei jeweils eine Beleuchtungseinrichtung 38, 40 zur Photooxidation des zu untersuchenden Mediums angeordnet. Die Anordnung der Beleuchtungseinrichtung 38, 40 erfolgt dabei derart, dass zumindest ein überwiegender Teil des Mediums mit dem durch die Beleuchtungseinrichtungen 38, 40 gebildeten Lichtstrahlen belichtet werden kann. Man erkennt, dass die Beleuchtungseinrichtungen 38, 40 an jeweils einer in den Zwischenraum zwischen den beiden Halteblockschenkeln 30, 32 zeigenden Oberfläche der Halteblockschenkel 30, 32 angeordnet sind.

Die Beleuchtungseinrichtungen 38, 40 umfassen dabei jeweils eine Vielzahl von LEDs, die auf entsprechenden Platinen 54, 56 angeordnet sind. Die Platinen 54, 56 sind an den genannten Oberflächen der Halteblockschenkel 30, 32 befestigt. Es besteht aber auch die Möglichkeit, dass die Halteblockschenkel 30, 32 entsprechende Ausnehmungen zur Aufnahme der Platinen 54, 56 aufweisen. Bei den genannten LEDs kann es sich insbesondere um Hochleistungs- LEDs handeln. Die LEDs oder Hochleistungs-LEDs müssen geeignet sein, Licht abzustrahlen, welches eine Photooxidation des in dem Probeaufnahmegefäß 12 aufgenommenen und zu untersuchenden Mediums bewirkt. In dem dargestellten Ausführungsbeispiel umfassen die Beleuchtungseinrichtungen 38, 40 jeweils zwischen 10 und 25 LEDs, wobei eine Lichtleistung pro LED zwischen 2,5 W und 3,5 W, insbesondere 3 W liegt. Zudem sind die in dem Ausführungsbeispiel gezeigten LEDs geeignet, Licht in einem Wellenlängenbereich von 450 nm bis 500 nm, insbesondere von 470 nm bis 480 nm, abzugeben.

Die Haltevorrichtung 10 weist zudem eine Transmissionsmessvorrichtung 42 zur Messung einer Transmission des auf das Medium gerichteten Anregungslichtstrahls 116, 118 der Anregungslichtquellen 80, 82 auf. Dabei ist die Transmissionsmessvorrichtung 42 an einer in den genannten Zwischenraum zeigenden Oberfläche des Basiselementes 28 des Halteblocks 26 angeordnet. Die Transmissionsmessvorrichtung 42 kann dabei als Fotodiode ausgebildet sein. Zudem ist die Transmissionsmessvorrichtung 42 datenleitend mit der Auswerteeinheit 112 verbunden. Mittels der Transmissionsmessvorrichtung 42 und der damit ermittelten Messwerte können beispielsweise unterschiedliche Trübungen des Mediums, insbesondere des hier zu untersuchenden Urins, ausgeglichen bzw. standardisiert werden.

Des Weiteren erkennt man, dass die in den Zwischenraum gerichteten Flächen der Halteblockschenkel 30, 32 zumindest teilweise einem Außenumfang 60 des aufzunehmenden Probeaufnahmegefäßes 12 angepasst sind. Zudem weisen die in den Zwischenraum gerichteten Flächen der Halteblockschenkel 30, 32 jeweils Führungsschienen 50, 52 zur Führung und Aufnahme des aufzunehmenden Probeaufnahmegefäßes 12 auf. Das Probenaufnahmegefäß 12 weist hierfür Ausnehmungen 62, 64 zur Führung der Führungsschienen 50, 52 auf. Zudem weist das Probenaufnahmegefäß 12 Vorsprünge 66 auf, die an den Innenumfang der Einführöffnung 36 angepasst sind. Der in den Figuren dargestellte Halteblock 26 besteht aus einem wärmeaufnehmenden Material, insbesondere aus Aluminium oder Edelstahl. Auch die Platinen 54, 56 können ein Trägermaterial aus Aluminium oder Kupfer aufweisen, was zu einer Wärmeabfuhr bzw. Wärmeaufnahme führt und die Lebensdauer der verwendeten LEDs verlängert. Des Weiteren erkennt man wiederum, dass die Platinen 54, 56 an der in den Zwischenraum zwischen den Halteblockschenkeln 30, 32 zeigenden Oberfläche der jeweiligen Halteblockschenkel 30, 32 befestigt sind. Die Platinen 54, 56 bzw. die Beleuchtungseinrichtungen 38, 40 werden im Übrigen über die Steuereinheit 110 gesteuert. Hierfür wird eine entsprechende Verbindung zwischen den Platinen 54, 56 und der Steuereinheit 10 nach einem Einführen bzw. Andocken der Haltevorrichtung 10 in bzw. an der Messvorrichtung 70 erzeugt.

**Figur 7** zeigt ein Diagramm zur Darstellung der Effizienz der Anzahl von Photooxidationszyklen bei der Belichtung der zu untersuchenden Urinprobe mittels der Beleuchtungseinrichtungen der Haltevorrichtung 10 der Messvorrichtung 70. Man erkennt, dass nach ca. 10 Belichtungszyklen die Fluoreszenzintensität nicht mehr signifikant zunimmt. Dies bedeutet, dass hier bereits eine nahezu vollständige Photooxidation der in der Urinprobe vorhandenen Porphyrinogene zu Porphyrin erfolgt ist.

## Patentansprüche

1. Haltevorrichtung (10) zur Aufnahme und Halterung von mindestens einem Probeaufnahmegefäß (12), wobei das Probeaufnahmegefäß (12) transparent und/oder transluzent ausgebildet ist und einen Aufnahmeraum (24) für ein zu untersuchendes, fließfähiges Medium aufweist, wobei die Haltevorrichtung (10)
- einen Halteblock (26) mit mindestens zwei, an einem Basiselement (28) angeordneten und parallel zueinander verlaufenden Halteblockschenkeln (30, 32) aufweist, wobei ein Zwischenraum zwischen den Halteblockschenkeln (30, 32) als Einführöffnung (36) für das Probeaufnahmegefäß (12) ausgebildet ist, und
- mindestens eine Beleuchtungseinrichtung (38, 40) zur Photooxidation des zu untersuchenden Mediums umfasst,
wobei die Beleuchtungseinrichtung (38, 40) an einer in den Zwischenraum zeigenden Oberfläche von mindestens einem der Halteblockschenkeln (30, 32) direkt oder indirekt angeordnet ist, und
ein dem Basiselement (28) des Halteblocks (26) gegenüberliegendes Ende des Zwischenraums als Lichteinlaßöffnung (34) für einen Anregungslichtstrahl (116, 118) ausgebildet ist.

2. Haltevorrichtung nach Anspruch 1, **dadurch gekennzeichnet, dass** die Beleuchtungseinrichtung (38, 40) mindestens eine LED, insbesondere eine Hochleistungs-LED, umfasst, wobei die LED oder Hochleistungs-LED geeignet ist, Licht abzustrahlen, welches eine Photooxidation des in dem Probeaufnahmegefäß (12) aufgenommenen und zu untersuchenden Mediums bewirkt.

3. Haltevorrichtung nach Anspruch 1 oder 2, **dadurch gekennzeichnet, dass** die LED oder Hochleistungs-LED geeignet ist, Licht in einem Wellenlängenbereich von 450 nm bis 500 nm, insbesondere von 470 nm bis 480 nm, abzugeben.

4. Haltevorrichtung nach einem der vorhergehenden Ansprüche, **dadurch gekennzeichnet, dass** die Haltevorrichtung (10) mindestens eine Transmissionsmessvorrichtung (42) zur Messung einer Transmission des auf das Medium gerichteten Anregungslichtstrahls (116, 118) mindestens einer Anregungslichtquelle (80, 82) aufweist, wobei die Transmissionsmessvorrichtung (42) an einer in den Zwischenraum zeigenden Oberfläche des Basiselements (28) des Halteblocks (26) direkt oder indirekt angeordnet ist

5. Haltevorrichtung nach einem der vorhergehenden Ansprüche, **dadurch gekennzeichnet, dass** die in den Zwischenraum gerichteten Flächen der Halteblockschenkel (30, 32) zumindest teilweise einem Außenumfang (60) des aufzunehmenden Probeaufnahmegefäßes (12) angepasst sind und/oder die in den Zwischenraum gerichteten Flächen der Halteblockschenkel (30, 32) Führungsschienen (50, 52) zur Führung und Aufnahme des aufzunehmenden Probeaufnahmegefäßes (12) aufweisen.

6. Haltevorrichtung nach einem der vorhergehenden Ansprüche, **dadurch gekennzeichnet, dass** der Halteblock (26) ganz oder teilweise aus einem wärmeaufnehmenden Material, insbesondere Aluminium oder Edelstahl, besteht und/oder
dass die Beleuchtungseinrichtung (38, 40) auf einer Platine (54, 56) angeordnet ist, wobei ein Trägermaterial der Platine (54, 56) aus Aluminium oder Kupfer besteht und/oder die Platine (54, 56) an der in den Zwischenraum zeigenden Oberfläche von mindestens einem der Halteblockschenkel (30, 32) befestigt ist.

7. Messvorrichtung zum quantitativen Bestimmen mindestens einer in einem fließfähigen Medium vorkommenden fluoreszierenden, lichtabsorbierenden und/oder lichtrückstreuenden Komponente umfassend
- ein Gehäuse (72);
- mindestens eine Haltevorrichtung (10) nach einem der vorhergehenden Ansprüche;
- mindestens eine Einführöffnung (74) in dem Gehäuse (72) zur Aufnahme der Haltevorrichtung (10);
- mindestens eine Lichtquelle (80, 82) zur Erzeugung mindestens eines Anregungslichtstrahls (116, 118) mit mindestens einer vorbestimmten Wellenlänge, wobei der Anregungslichtstrahl (116, 118) über ein optisches System (84) zu mindestens einem im Bereich der Einführöffnung (74) angeordneten Lichtauslaß (114) und der damit lichtleiten verbundenen Lichteinlaßöffnung (34) der Haltevorrichtung (10) leitbar ist;
- mindestens eine Steuereinrichtung (110) zur Steuerung einer Belichtungszeit und/oder einem Belichtungsintervall des Anregungslichtstrahls (116, 118) und/oder einer Belichtungszeit und/oder einem Belichtungsintervall des von der Beleuchtungseinrichtung (38, 40) erzeugten Lichtstrahls, und
- mindestens eine Auswerteeinrichtung (106, 112) zum Empfang und quantitativen Auswertung der durch den Anregungslichtstrahl (116, 118) in dem zu analysierenden Medium erzeugten Absorption, Rückstreuung und/oder Fluoreszenz.

8. Messvorrichtung nach Anspruch 7, **dadurch gekennzeichnet, dass** das Medium Urin ist und/oder das die zu analysierende Komponente aus der Gruppe der Porphyrine gewählt ist.

9. Messvorrichtung nach Anspruch 7 oder 8, **dadurch gekennzeichnet, dass** die Messvorrichtung (70) mindestens eine Bedien- und/oder Anzeigevorrichtung (78) umfasst.

10. Messvorrichtung nach einem der Ansprüche 7 bis 9, **dadurch gekennzeichnet, dass** ein Detektionslichtstrahl (120) über die Lichteinlaßöffnung (34) der Haltevorrichtung (10) und den Lichtauslaß (114) des Gehäuses (72) zu der Auswerteeinrichtung (106) geleitet wird.

11. Messvorrichtung nach einem der Ansprüche 7 bis 10, **dadurch gekennzeichnet, dass** die Messvorrichtung (70) mindestens eine erste Anregungslichtquelle (80), insbesondere Laserlichtquelle, umfasst, wobei die Anregungslichtquelle (80) einen ersten Anregungslichtstrahl (116) innerhalb eines Wellenlängenbereichs von 385 nm bis 410 nm, insbesondere von 400 nm bis 401 nm, erzeugt oder
die Messvorrichtung (70) mindestens eine erste Anregungslichtquelle (80), insbesondere Laserlichtquelle, umfasst, wobei die Anregungslichtquelle (80) einen ersten Anregungslichtstrahl (116) innerhalb eines Wellenlängenbereichs von 385 nm bis 410 nm, insbesondere von 400 nm bis 401 nm, erzeugt und eine zweite Anregungslichtquelle (82), insbesondere Laserlichtquelle, umfasst, wobei die zweite Anregungslichtquelle (82) einen zweiten Anregungslichtstrahl (118) mit einer von dem Wellenlängenbereich des ersten Anregungslichtstrahls (116) unterschiedlichen Wellenlängenbereich erzeugt.

12. Messvorrichtung nach einem der Ansprüche 7 bis 11, **dadurch gekennzeichnet, dass** die Transmissionsmessvorrichtung (42) mit der der Auswerteeinrichtung (112) und/oder Steuereinrichtung (110) datenleitend verbunden ist.

13. Messvorrichtung nach einem der Ansprüche 7 bis 12, **dadurch gekennzeichnet, dass** die Steuereinrichtung (110) die Beleuchtungseinrichtung (38, 40) derart steuert, dass mit dem erzeugten Lichtstrahl
- eine Belichtung und Photooxidation des zu untersuchenden Mediums vor einer Bestrahlung mit dem Anregungslichtstrahl (116, 118), oder
- eine Belichtung und Photooxidation des zu untersuchenden Mediums nach einer Bestrahlung mit dem Anregungslichtstrahl (116, 118), oder
- eine Belichtung und Photooxidation des zu untersuchenden Mediums vor und nach einer Bestrahlung mit dem Anregungslichtstrahl (116, 118),
erfolgt.

14. Messvorrichtung nach einem der Ansprüche 7 bis 13, **dadurch gekennzeichnet, dass** die Steuereinrichtung (110) die Beleuchtungseinrichtung (38, 40) und die Anregungslichtquelle (80, 82) derart steuert, dass die Anzahl der Mess-/Photooxidationszyklen in dem zu untersuchenden Medium zwischen 1 und 40 liegt und/oder
dass die Steuereinrichtung (110) die Beleuchtungseinrichtung (38, 40) derart steuert, dass eine Belichtungszeit für die Photooxidation zwischen 50 ms und 500 ms, insbesondere 150 ms, beträgt und/oder
die Steuereinrichtung (110) die Anregungslichtquelle (80, 82) derart steuert, dass eine Bestrahlung des zu untersuchenden Mediums mit dem Anregungslichtstrahl (116, 118) zwischen 5 ms und 50 ms, insbesondere für 15 ms, erfolgt und/oder
die Steuereinrichtung (110) und/oder die Auswerteeinheit (112) in der Bedien- und Anzeigevorrichtung (78) integriert sind.

15. Messvorrichtung nach einem der Ansprüche 7 bis 14, **dadurch gekennzeichnet, dass** die Beleuchtungseinrichtung (38, 40) zwischen 10 und 25 LEDs umfasst, wobei eine Lichtleistung pro LED zwischen 1,0 W und 3,5 W, insbesondere 3 W, beträgt.

16. Messvorrichtung nach einem der Ansprüche 7 bis 15, **dadurch gekennzeichnet, dass** optische System (84) den Anregungslichtstrahl (116, 118) der Anregungslichtquelle (80, 82) sowie einen von dem Medium rückgestreuten und/oder emittierten Detektionslichtstrahl (120) über Wegsamkeiten innerhalb der Messvorrichtung (70) leitet.
